# EUROPEAN PATENT SPECIFICATION

(11) **EP 3 693 977 B1**
(45) Date of publication and mention of the grant of the patent: **08.12.2021**
(21) Application number: 18865891.8
(22) Date of filing: 11.10.2018
(51) Int. Cl.: G21F 3/00, G21F 1/08, A61N 5/10, G21F 1/10

(54) **SHIELD ADHESIVE HAVING NEUTRON SHIELDING PROPERTIES**
ABSCHIRMUNGSKLEBER MIT NEUTRONENABSCHIRMENDEN EIGENSCHAFTEN
ADHÉSIF DE BLINDAGE AYANT DES PROPRIÉTÉS DE BLINDAGE NEUTRONIQUE

(30) Priority: 11.10.2017 JP 2017198061
(43) Date of publication of application: 12.08.2020
(73) Proprietor: Nippon Light Metal Company, Ltd., Tokyo 1050004 (JP)
(72) Inventor: USUI, Hideaki, Tokyo 105-0004 (JP); FURUYA, Hidaka, Inazawa-shi Aichi 492-8144 (JP); TAKAHASHI, Maki, Tokyo 105-0004 (JP); ISHIKAWA, Hidenori, Tokyo 105-0004 (JP)
(74) Representative: Plasseraud IP
(86) International application number: PCT/JP2018/038006
(87) International publication number: WO 2019/074079

(56) References cited:
- JP-A- S5 194 098
- JP-A- H08 201 581
- JP-A- 2016 211 968
- US-A1- 2013 112 898
- US-A1- 2014 265 057
- US-A1- 2016 284 430
- US-A1- 2017 052 265

## Description

### TECHNICAL FIELD

The present invention relates to a shield adhesive having neutron-shielding performance.

### BACKGROUND ART

In recent years, research and development of boron-neutron capture therapy (BNCT) is rapidly progressing as a means of cancer treatment. Boron-neutron capture therapy is radiotherapy which uses a neutron beam. First, a boron compound that is specifically taken in by cancer cells is administered to a patient. Subsequently, the cancer cells in which the boron compound has accumulated are irradiated with a neutron beam whose energy is controlled within a predetermined range. When the neutron beam collides with the boron compound, α rays are generated. The cancer cells are killed by the α rays.

Boron-neutron capture therapy is promising as a means of treating cancer and is moving into the clinical trials phase. The neutron irradiation apparatus used in boron-neutron capture therapy brings about therapeutic effects by using a thermal neutron beam or an epithermal neutron beam. The neutron irradiation environment is a field where radioactive rays having energies in a certain range coexist.

It has been necessary so far to use a nuclear reactor as a neutron generator for supplying a neutron beam to a neutron irradiation apparatus. However, in recent years, small neutron generators to be installed in hospitals are being proposed. In such a small neutron generator, protons and deuterons accelerated by an accelerator collide with a beryllium or lithium target. The generated neutron beam has a higher proportion of thermal neutrons and epithermal neutrons than those generated in conventional equipment. The generated neutron beam is then decelerated by a moderator to provide a neutron beam irradiation environment having little influence on human bodies.

When neutrons are irradiated in neutron capture therapy, it is necessary to provide a neutron-shielding means to irradiate a specific site. In order to examine the effects of neutron capture therapy, experiments of irradiating small animals, such as mice, with neutrons have been performed. Patent Document 1 relates to a case of applying neutron capture therapy using a shielding plate of lithium fluoride to mammals other than human beings. The object thereof is to minimize neutrons given to normal tissue when the target site is deep inside the irradiation object, and to provide sufficient neutrons to the target located deep inside the irradiation object by suppressing the reduction in depth and reachability of neutrons going into the body.

Patent Document 1: Japanese Unexamined Patent Application, Publication No. 2004-233168

### DISCLOSURE OF THE INVENTION

### Problems to be Solved by the Invention

However, in these shielding methods, the installability and shielding properties of a shield body may be restricted by the shape of the organism which is to be an irradiation object, as well as the size of the irradiation site. Accordingly, there is required a shielding means that three-dimensionally shields the irradiation object and separates an irradiation region of the neutron beam from a non-irradiation region by means of a simple structure.

Another shielding structure is known from patent document JP H08201581 A.

As the shielding means, a box-type structure having neutron beam-shielding performance can be conceived, which can accommodate an organism, i.e., an irradiation object. Such a box-type structure can be produced by combining and joining a plurality of shielding plates. In the joining, an adhesive can be used. However, a neutron beam coming from the outside by an irradiation passes through the joining portion and enters the inside. Accordingly, it is desirable to impart a function to shield the joining portion from neutrons. Thus, it is an object of the present invention to provide an adhesive having shielding properties that can impart neutron-shielding properties to the joining portion of a structure having neutron-shielding performance.

### Means for Solving the Problems

The present inventors have considered how to solve the above-described problems, and as a result, the present invention provides a shielding adhesive according to claim 1 and the use of that adhesive for repairing a defective part of shielding plates made of a lithium fluoride sintered body or for filling a gap at a joining portion of the shielding plates, according to claim 6.

Preferred embodiments are defined in the dependent claims.

### Effects of the Invention

A box-type structure having a simple three-dimensional structure can be provided by applying a shielding adhesive according to the present invention for joining a plurality of shielding plates to each other. Since the shielding adhesive according to the present invention can impart neutron-shielding to the site joined with the adhesive, the neutron-shielding performance of the entire structure can be improved.

### BRIEF DESCRIPTION OF THE DRAWINGS

[Fig. 1] Fig. 1 is a schematic view illustrating an embodiment of a box-type structure to which a shielding adhesive is applied.
[Fig. 2] Fig. 2 includes schematic views illustrating another embodiment of a box-type structure to which a shielding adhesive is applied. (a) is a front view, and (b) is a perspective view.
[Fig. 3] Fig. 3 includes schematic views for explaining joining structures of shielding plates to which a shielding adhesive is applied (a) is a view illustrating a joining structure when the edge portions of shielding plates have flat joining faces in a thickness direction. (b) is a view illustrating a joining structure when the edge portions of shielding plates have inclined joining faces. (c) is a view illustrating a joining structure when the edge portions of shielding plates have concave and convex joining faces.
[Fig. 4] Fig. 4 includes views illustrating an exemplary form of a shielding plate to which a shielding adhesive is applied. (a) is a perspective view, (b) is a plan view, (c) is a cross-sectional view along the line A-A, and (d) is a cross-sectional view along the line B-B.
[Fig. 5] Fig. 5 includes views illustrating another exemplary form of a shielding plate to which a shielding adhesive is applied. (a) is a perspective view, (b) is a plan view, (c) is a cross-sectional view along the line A-A, and (d) is a cross-sectional view along the line B-B.
[Fig. 6] Fig. 6 includes views illustrating another exemplary form of a shielding plate to which a shielding adhesive is applied. (a) is a perspective view, (b) is a plan view, (c) is a cross-sectional view along the line A-A, and (d) is a cross-sectional view along the line B-B.
[Fig. 7] Fig. 7 includes views illustrating another exemplary form of a shielding plate to which a shielding adhesive is applied. (a) is a perspective view, (b) is a plan view, (c) is a cross-sectional view along the line A-A, and (d) is a cross-sectional view along the line B-B.
[Fig. 8] Fig. 8 includes views illustrating another exemplary form of a shielding plate to which a shielding adhesive is applied. (a) is a perspective view, (b) is a plan view, (c) is a cross-sectional view along the line A-A, and (d) is a cross-sectional view along the line B-B.
[Fig. 9] Fig. 9 includes views illustrating another exemplary form of a shielding plate to which a shielding adhesive is applied. (a) is a perspective view, (b) is a plan view, (c) is a cross-sectional view along the line A-A, and (d) is a cross-sectional view along the line B-B.
[Fig. 10] Fig. 10 includes views illustrating another embodiment related to the box-type structure to which a shielding adhesive is applied. (a) is a perspective view, (b) is a front view, (c) is a right-side view. (d) is a cross-sectional view along the line A-A, (e) is a cross-sectional view along the line B-B, (f) is a cross-sectional view along the line C-C, and (g) is a view illustrating the internal structure.
[Fig. 11] Fig. 11 includes views illustrating another embodiment related to the box-type structure to which a shielding adhesive is applied. (a) is a perspective view, (b) is a perspective view from another direction, (c) is a front view, (d) is a plan view, (e) is a right-side view. (f) is a cross-sectional view along the line A-A, (g) is a cross-sectional view along the line B-B, and (h) is a cross-sectional view along the line C-C.
[Fig. 12] Fig. 12 includes views illustrating another embodiment related to the box-type structure to which a shielding adhesive is applied. (a) is a perspective view, and (b) is a perspective view from another direction.
[Fig. 13] Fig. 13 includes views illustrating another embodiment related to the box-type structure to which a shielding adhesive is applied. (a) is a perspective view, and (b) is a perspective view from another direction.
[Fig. 14] Fig. 14 includes views illustrating another embodiment related to the box-type structure to which a shielding adhesive is applied. (a) is a perspective view, and (b) is a perspective view from another direction.
[Fig. 15] Fig. 15 is a perspective view illustrating another embodiment related to the box-type structure to which a shielding adhesive is applied.
[Fig. 16] Fig. 16 is a view illustrating another exemplary form of a shielding plate to which a shielding adhesive is applied.
[Fig. 17] Fig. 17 includes schematic views for explaining joining structures of shielding plates to which a shielding adhesive is applied. (a) is a view illustrating a joining structure including edge portions having flat joining faces. (b) is a view illustrating a joining structure including edge portions having inclined joining surfaces. (c) is a view illustrating a joining structure including edge portions having concave and convex joining surfaces.
[Fig. 18] Fig. 18 includes views for explaining an embodiment of applying a shielding adhesive for repairing a shielding plate. (a) shows an exemplary application for repairing a corner of a shielding plate. (b) shows an examplary application for repairing a surface of a shielding plate. (c) shows an examplary application for filling a gap of the joining portion of shielding plates.
[Fig. 19] Fig. 19 is a view for explaining a box-type structure of an Example to which a shielding adhesive is applied.

### PREFERRED MODE FOR CARRYING OUT THE INVENTION

Embodiments of the shielding adhesive according to the present invention will now be described. The present invention is not limited to the following embodiments. The scope of the invention is defined by the appended claims.

### (Shielding adhesive)

The shielding adhesive according to the present invention is a shielding adhesive having neutron-shielding performance. Edge portions of shielding plates are preferably joined by the shielding adhesive. The joining structure of edge portions can be strengthened by applying the shielding adhesive to a face (end face) of the edge portions of shielding plates and abutting the edge portions against each other. In addition, it is possible to temporarily fix a part of the end faces of shielding plates with adhesive tape, and then fix permanently fix a part of the end faces with an adhesive. In this case, the temporarily fixed face can be used as a removable opening surface.

The shielding adhesive according to the present invention preferably contains a lithium fluoride powder in an amount of 30 wt% or more. Such an adhesive functions as a sealant filling the gap between the shielding plates, as well as glueing shielding plates together. The adhesive incorporates lithium fluoride containing 6Li, which has a neutron-shielding function, has the effect of preventing a neutron beam from entering through the gap between the shielding plates.

When the viscosity of the adhesive is low, the adhesive may contain a lithium fluoride powder in an amount of preferably 40 wt% or more, more preferably 50 wt% or more, and further preferably 60 wt% or more in order for adjusting the viscosity to have appropriate application workability gap filling properties, and further enhanced neutron-shielding performance.

The shielding adhesive according to the present invention contains lithium fluoride in an adhesive, the main component of which is an epoxy resin that does not contain, or contains a very small amount of, an inorganic filler. Since epoxy resin has a low content of elements other than carbon, hydrogen, and oxygen, the radioactivation rate is low when irradiated with a neutron beam. Accordingly, it is suitable for use in medical fields such as radiotherapy. In addition, a two-liquid curing type epoxy resin is cured by mixing a main agent and a curing agent. Accordingly, when a powder is added, the main agent and the curing agent can be separately added to and kneaded with the powder and can be then mixed for reaction and curing. Thus, the kneading time has a margin, and the workability is improved. It is possible to select an adhesive having an appropriate working time, curing time and viscosity. If the viscosity is too low, the adhesive is apt to flow out of the applied gap, and the gap filling property is reduced. Accordingly, it is preferable to use an adhesive having an appropriately high viscosity. A short curing time is problematic because the time margin for assembling shielding plates into a desired form is reduced. A long curing time is problematic because it requires an elongated time for maintaining the shape of the structure, and therefore, temporary fixing must be performed for a long time.

### (Box-type structure)

The box-type structure to which the shielding adhesive according to the present invention is applied includes a plurality of shielding plates containing lithium fluoride, which has neutron-shielding performance Edge portions of the shielding plates are abutted and joined to each other. Here, the term "neutron shielding performance" refers to the performance of shielding neutron beams. In the present specification, "neutron shielding" may be described as "neutron beam shielding". The shielding plate to which the shielding adhesive according to the present invention is applied means a neutron shielding plate. The box-type structure is composed of m a plurality of shielding plates having neutron shielding performance assembled into a box-like shape, and has a simple three-dimensional structure.

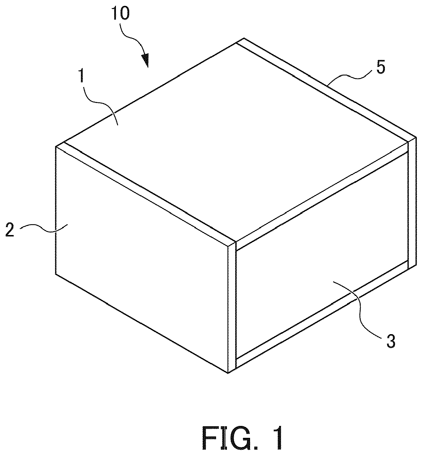

The external appearance of the box-type structure is shown in Fig. 1. Six shielding plates, e.g., shielding plates 1 and 2, are assembled to make the box-type structure 10. The edge portion of the shielding plates are abutted on each other such that the end faces are closely joined to each other by means of the shielding adhesive according to the present invention. The box-type structure shown in Fig. 1 is an embodiment in which all outer faces are surrounded by shielding plates.

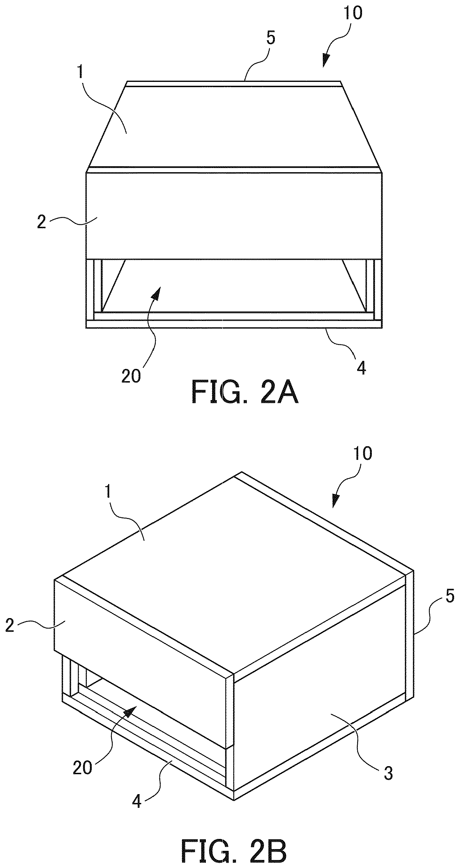

The box-type structure 10 shown by (a) and (b) in Fig. 2 is another embodiment having an opening 20 provided in a part of the outer faces. Six shielding plates, e.g., shielding plates, 1, 2, 3, and 4, are assembled to make the box-type structure. The edge portions of the shielding plates are abutted on each other such that the end faces of the shielding plates are closely joined to each other. As shown by (a) and (b) in Fig. 2, a part of the sides of the shield 2 is short in length such that the opening 20 is formed at that part.

The shielding plates of the box-type structure are made of a material containing lithium fluoride (LiF), which is excellent in its neutron-shielding performance. The shielding plates of the box-type structure prevent the neutron beam radiated from the outside from passing through. Accordingly, the shielding plates reduce the neutron beams that reach the inside of the box-type structure, and thus can make a region (non-irradiation region) that is not substantially irradiated with the neutron beam in the box-type structure.

The box-type structure has a three-dimensional shape, such as a cube or a rectangular parallelepiped, which forms a three-dimensional neutron beam-shielding space. The shielding space can accommodate a small animal, even if it has a certain volume or more.

Since the edge portions of the shielding plates are joined to each other with the shielding adhesive, a neutron beam is sufficiently prevented from passing through the gap between the edge portions. Accordingly, a neutron beam-shielding space can be formed inside the box-type structure.

In the box-type structure having neutron-shielding performance, since a plurality of shielding plates having neutron-shielding performance are joined in combination, a simple three-dimensional structure can be obtained. The size of the box-type structure can be freely adjusted according to the size and the number of the combined shielding plates.

In addition, the edge portions of the neutron-shielding plates can be easily joined to each other by providing stepped or inclined joining structures at the edge portions of the neutron-shielding plates, and thus an effect of stabilizing the joining structure can be obtained.

It is possible to accommodate an organism or the like in a space shielded from a neutron beam and to perform a test in a state in which not all of the organism or the like is irradiated with a neutron beam. Furthermore, when the box-type structure has a partly open structure, a part of the organism or the like can be taken outside of the shielding region and can be irradiated with a neutron beam in only that part, broadening the application range of neutron beams in irradiation objects and test conditions.

This box-type structure is suitable for the field of radiating a neutron beam and examining its action and influence. It can be used in a test using a small animal such as a mouse, or in an irradiation test relating to radiotherapy, etc. In addition, a plurality of irradiation bodies can be simultaneously irradiated by providing, for example, partitions in the box-type structure or by providing a plurality of the box-type structures, which improves the efficiency of an irradiation test.

### (Shielding plates)

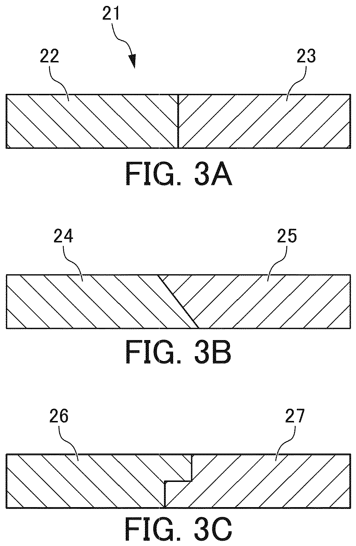

The shielding adhesive according to the present invention can be applied to shielding plates 22 and 23 including edge portions having flat joining faces in the thickness direction as shown by (a) in Fig. 3. In addition, the shielding adhesive can be applied to shielding plates 24 and 25 including edge portions having inclined joining faces as shown by (b) in Fig. 3. In these shielding plates, the edge portions are abutted against each other, and the joining surfaces are fixed to each other with a shielding adhesive. Accordingly, firm engagement and sufficient neutron-shielding performance are imparted to the assembly of the shielding plates.

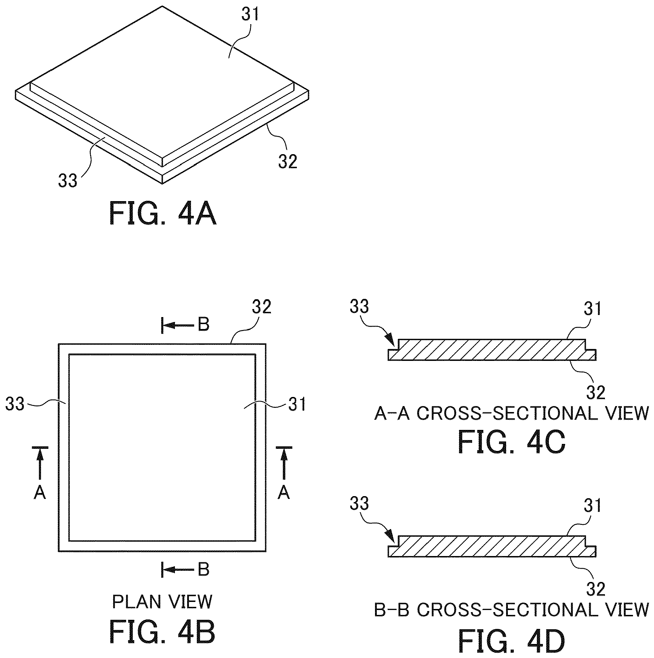

Furthermore, the shielding adhesive can be applied to the shielding plates 26 and 27 including edge portions having stepped joining faces as shown by (c) in Fig. 3. A concave portion (hereinafter, referred to as "concave") or a convex portion (hereinafter, referred to as "convex") is provided at the edge portions of the shielding plates 26 and 27 in a stepped form. The concave and the convex make a close fit together. The joining faces are fixed with a shielding adhesive. Regarding the joining structure in which a shielding adhesive is applied to a shielding plate having such a stepped joining surface, there are various exemplary forms as shown in Figs. 4 to 9.

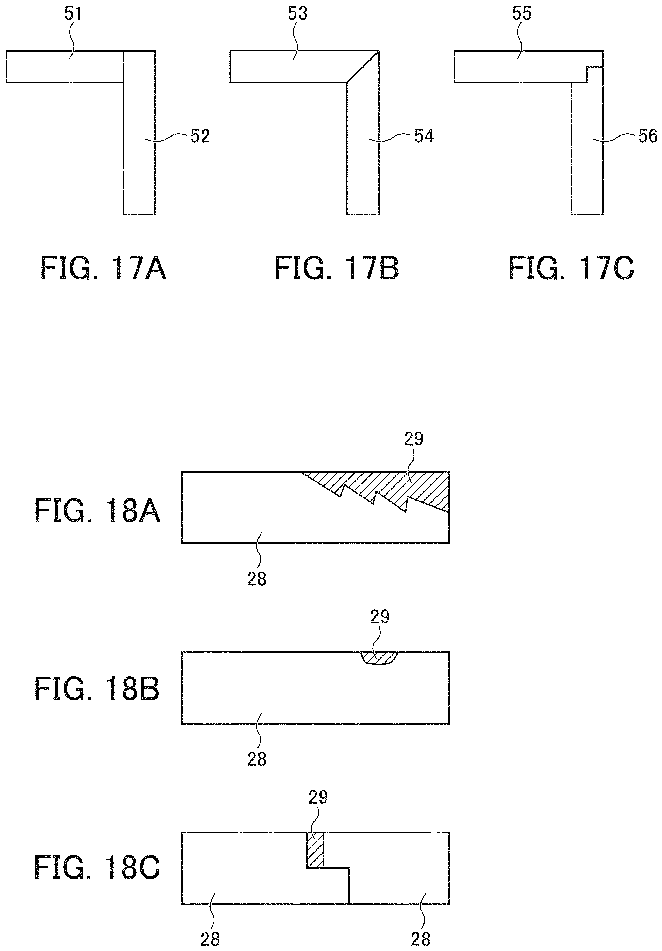

(a) to (c) in Fig. 17 show exemplary forms in which joining structures of box-type structures are formed by using these shielding plates. (a) in Fig. 17 is a view illustrating a joining structure in which a shielding adhesive is applied to shielding plates having flat joining faces. (b) in Fig. 17 is a view illustrating a joining structure in which a shielding adhesive is applied to shielding plates having inclined joining faces. (c) in Fig. 17 is a view illustrating a joining structure in which a shielding adhesive is applied to joining faces having stepped concave and convex portions.

The shielding adhesive according to the present invention can be applied for repair when a part of a shielding plate is chipped off. For example, (a) in Fig. 18 shows an example of a shield body having a chipped portion 29 at a corner of a shielding plate 28. (b) in Fig. 18 shows an example of a shield body having a chipped portion 29 on a surface of a shielding plate 28. In each of the shield bodies 28, the chipped off portion or a part having the same shape as that of the chipped off portion is joined to the body of the shielding plate by means of a shielding adhesive to restore the shielding plate to the original shape. (c) in Fig. 18 shows an example of using the shielding adhesive in combination with a neutron beam sealant as a filler for structural strengthening when the gap between the halving joint portions of the shielding plates is relatively large.

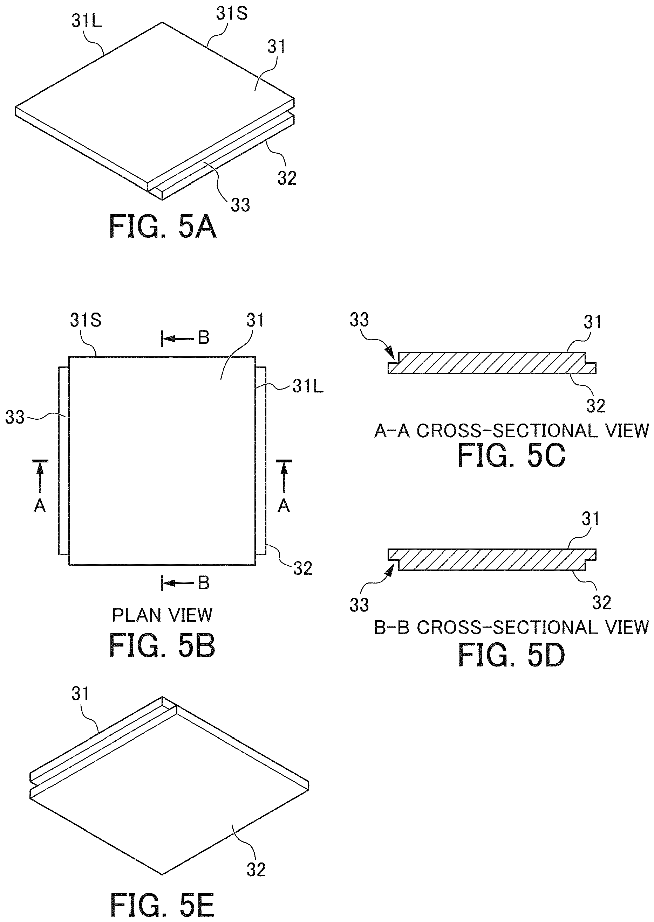

(a) to (d) in Fig. 4 are views illustrating exemplary forms relating to shielding plates to which a shielding adhesive according to the present invention is applied. (a) in Fig. 4 is a perspective view, (b) in Fig. 4 is a plan view, (c) in Fig. 4 is a cross-sectional view along the line A-A, and (d) in Fig. 4 is a cross-sectional view along the line B-B. Figs. 5 to 9 show other exemplary forms of shielding plates. In each figure, similar to (a) to (d) of Fig. 4, (a) is a perspective view, (b) is a plan view, (c) is a cross-sectional view along the line A-A, and (d) is a cross-sectional view along the line B-B.

Fig. 4 shows an example of a shielding plate including edge portions having stepped joining faces at four sides of the shielding plate. As understood from the cross-sectional views shown by (c) and (d) in Fig. 4, the lengths of the four sides of the shielding plate on the upper side 31 are all shorter in length than those of the four sides on the lower side 32. As a result, as shown by (a) in Fig. 4, this shielding plate has a shape including steps 33 formed at the edge portions of four sides.

Fig. 5 shows another example of a shielding plate including edge portions having stepped joining faces at four sides of the shielding plate. As understood from the cross-sectional views shown by (c) and (d) in Fig. 5, the upper side and the lower side of the shielding plate differ from each other in the lengths of the four sides. The two sides 31S among the four sides on the upper side 31 are shorter than the corresponding two sides on the lower side 32, while the other two sides 31L on the upper side are longer than the corresponding two sides on the lower side 32. As a result, as shown by (a) in Fig. 5, this shielding plate has a shape including steps 33 formed at the edge portions of four sides.

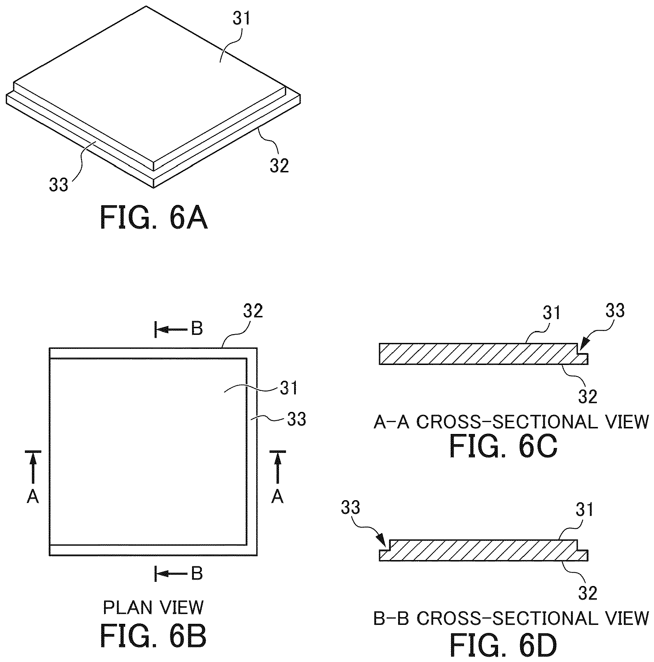

Fig. 6 shows an example of a shielding plate including edge portions having stepped joining faces at three sides of the shielding plate. As understood from the cross-sectional views shown by (c) and (d) in Fig. 6, three sides among the four sides on the upper side of the shielding plate are shorter in length than the corresponding three sides on the lower side. As a result, as shown by (a) in Fig. 6, this shielding plate has a shape including steps formed at the edge portions of the three sides.

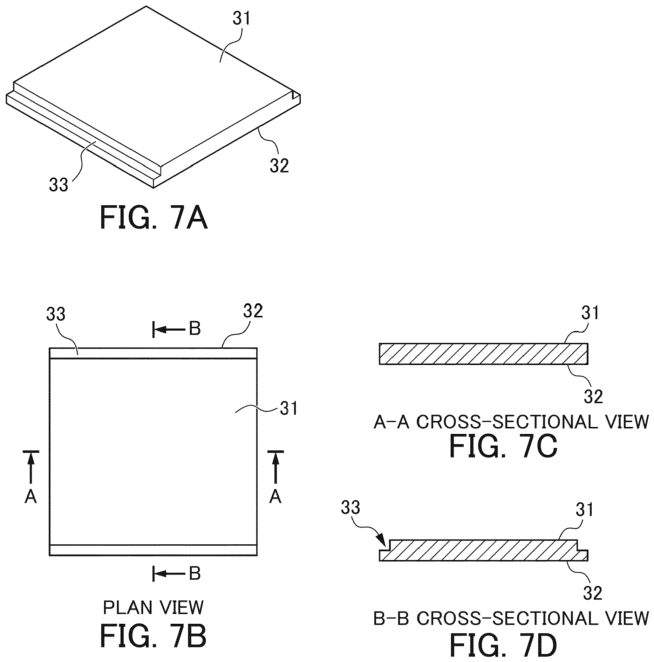

Fig. 7 shows an example of a shielding plate including edge portions having stepped joining faces at two sides of the shielding plate. As understood from the cross-sectional views shown by (c) and (d) in Fig. 7, two sides among the four sides on the upper side 31 of a shielding plate are shorter in length than the corresponding two sides on the lower side 32. As a result, as shown by (a) in Fig. 7, this shielding plate has a shape including steps 33 formed at the edge portions of two sides.

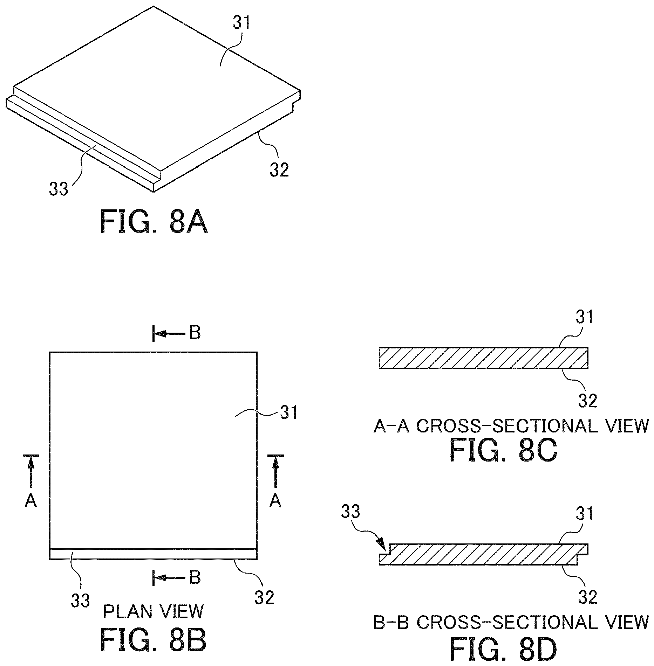

Fig. 8 shows another example of a shielding plate including edge portions having stepped joining faces at two sides of the shielding plate. As understood from the cross-sectional views shown by (c) and (d) in Fig. 8, the four sides on each of the upper side 31 and the lower side 32 of the shielding plate have the same length. However, two sides among the four sides are arranged at different positions from the corresponding two sides. As a result, as shown by (a) in Fig. 8, this shielding plate has a shape including steps 33 formed at the edge portions of two sides.

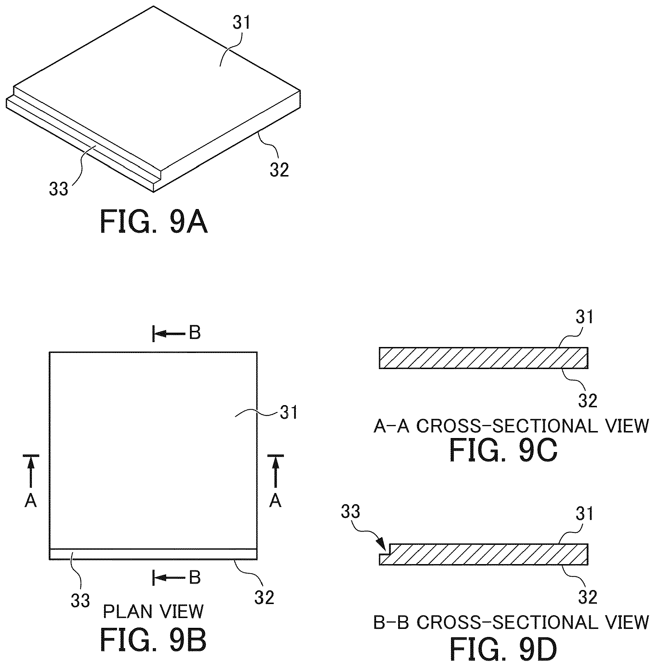

Fig. 9 shows an example of a shielding plate including edge portions having a stepped joining face at one side of the shielding plate. As understood from the cross-sectional views shown by (c) and (d) in Fig. 9, one side among the four sides on the upper side 31 of a shielding plate is shorter in length than the corresponding one side on the lower side 32. As a result, as shown by (a) in Fig. 9, this shielding plate has a shape including a step 33 formed at the edge portion of one side.

The shielding plates are not limited to those including the above-described edge portions having stepped joining surfaces. The shielding plate may include an edge portion having an inclined joining face as shown by (b) in Fig. 3 and may similarly have a shape including an inclined joining face formed at each edge portion of four sides, three sides, two sides, or one side.

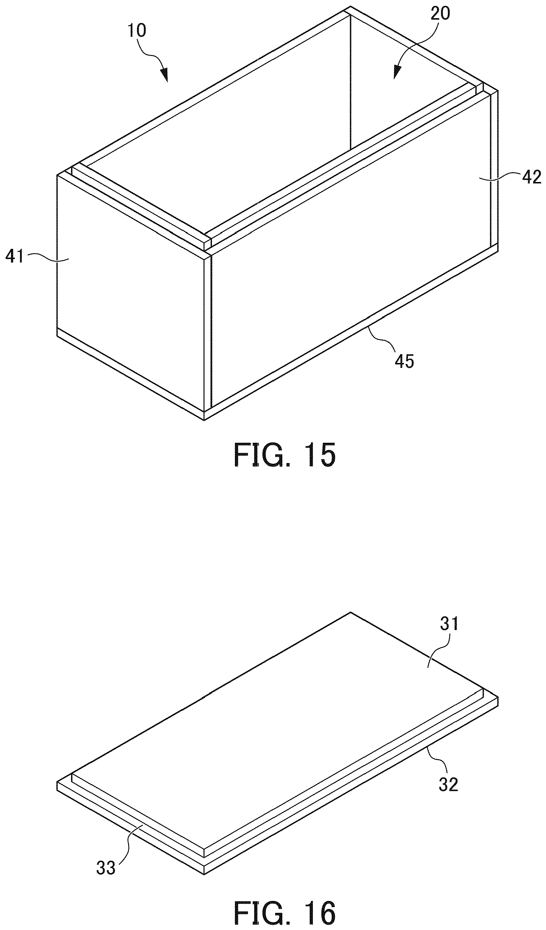

The planar shapes of the shielding plates shown in Figs. 4 to 9 are each a square, but the shape is not limited thereto and may be a rectangle as shown in Fig. 16.

A neutron beam radiated from the outside may pass through the shielding plates and the joining portion. For example, when the joining portion as shown by (a) in Fig. 3 includes an even joining surface, the neutron beam that has entered the joining portion may pass therethrough and enter the inside of the structure. When the joining portion includes inclined or concave and convex joining faces as shown by (b) or (c) in Fig. 3, the neutron beam that has passed through the shield body may pass through the joining portion and enter the inside. In contrast, the shielding adhesive according to the present invention imparts sufficient neutron-shielding properties to the joining portion. Accordingly, it is possible to prevent the neutron beam from passing through the joining portion, and to obtain an effect of improved shielding properties of the entire structure composed of shielding plates.

In addition, the structure composed of shields is stably fixed by joining the edge portions of the shielding plates with a shielding adhesive, causing an effect of increasing mechanical strength.

### (Face of box-type structure)

The box-type structure to which the shielding adhesive according to the present invention is applied has a plurality of faces, and at least one of the faces is preferably removable. The box-type structure is three-dimensionally configured and therefore has a plurality of outer faces. These faces have a removable structure. Accordingly, it is convenient for disassembling the box-type structure in, for example, preparation for an irradiation test or withdrawal after the test. In addition, the organism, i.e., an irradiation object, can be easily put in and taken out.

The box-type structure can be provided with an opening portion in a part of the faces. Since the outside of the box-type structure is in an environment under irradiation with a neutron beam, it is possible to expose a part of the irradiation object to the outside through the opening portion and to irradiate the part exclusively. For example, in order to irradiate the leg of a mouse exclusively for a test with a neutron beam, it is possible to three-dimensionally cover the mouse body with the box-type structure and place only the leg of the mouse in the irradiation environment outside the box-type structure.

### (Joining with adhesive tape)

The shielding plates may be fixed with adhesive tape in addition to the joining with a shielding adhesive. Alternatively, the edge portions of the shielding plates are abutted against each other, and the shielding plate surfaces are then attached to adhesive tape to fix the shielding plates. This example, however, is not part of the present invention . Thus, a box-type structure can be temporarily assembled.

The box-type structure fixed with adhesive tape can be disassembled by peeling off the adhesive tape after being used in a necessary test or can be reassembled. The adhesive tape may be any known product without specific limitation. In order to minimize the radioactivation rate by irradiation with a neutron beam, it is preferable to select colorless transparent or translucent tape free from coloring components and inorganic fillers in the tape base. The adhesive component adhering to the tape base surface is not particularly limited and may be, for example, a rubber or acrylic adhesive agent. The material of the tape base is not particularly limited and may be, for example, cellophane or acetate.

### (Embodiment of box-type structure)

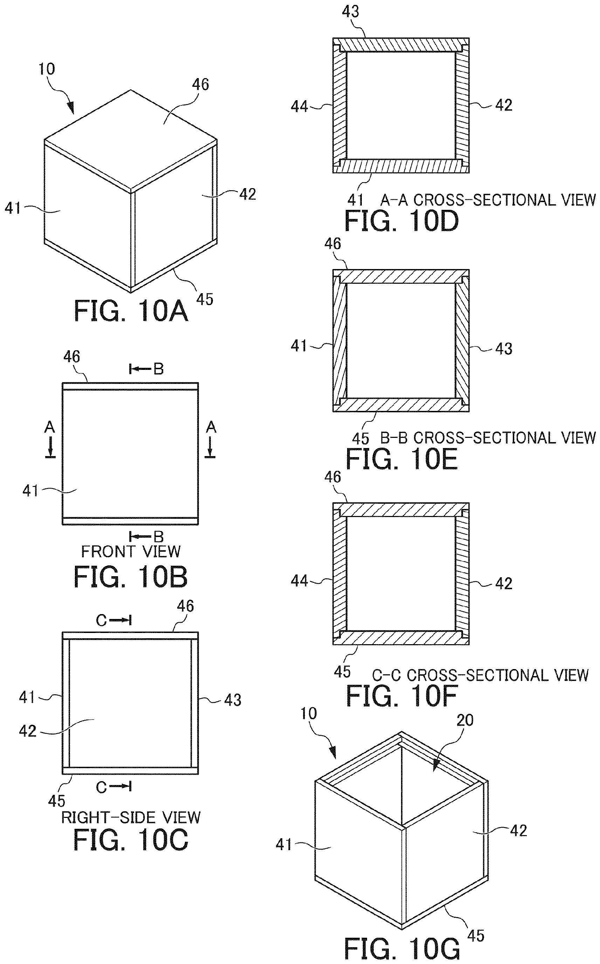

The box-type structure to which a shielding adhesive according to the present invention is applied can be provided in various shapes and configurations by combining the above-described shielding plates. Exemplary forms thereof are shown in Figs. 10 to 15.

(a) to (g) in Fig. 10 show embodiments related to the box-type structure. (a) in Fig. 10 is a perspective view, (b) in Fig. 10 is a front view, and (c) in Fig. 10 is a right-side view. (d) in Fig. 10 is a cross-sectional view along the line A-A, (e) in Fig. 10 is a cross-sectional view along the line B-B, and (f) in Fig. 10 is a cross-sectional view along the line C-C. (g) in Fig. 10 is a view illustrating the inside of the box-type structure when the upper shielding plate 46 is detached. As shown by (a) to (c) in Fig. 10, it is possible to provide a box-type structure composed of a combination of six shielding plates 41, 42, 43, 44, 45, and 46 that has a structure in which the shielding plates enclose the internal space completely. The shielding plates 41, 42, 43, 44, 45, and 46 have a structure including the edge portions abutted and joined via the joining portions. On the inside of the box-type structure, the stepped joining structure has a concave on the inside, as shown by (b) in Fig. 10.

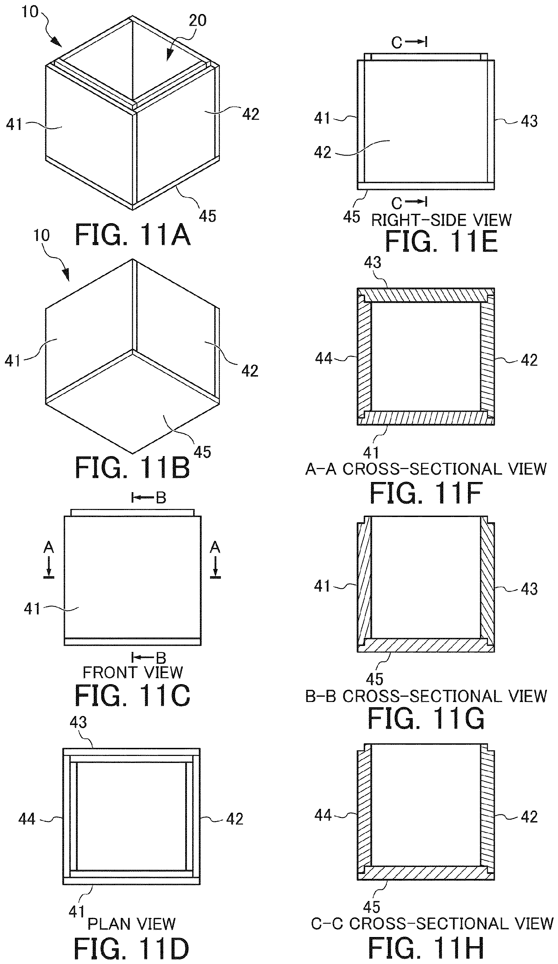

(a) to (h) in Fig. 11 show another embodiment related to the box-type structure to which a shielding adhesive is applied. (a) in Fig. 11 is a perspective view, (b) in Fig. 11 is a perspective view from another direction, and (c) in Fig. 11 is a front view. (d) in Fig. 11 is a plan view, and (e) in Fig. 11 is a right-side view. (f) in Fig. 11 is a cross-sectional view along the line A-A, (g) in Fig. 11 is a cross-sectional view along the line B-B, and (h) in Fig. 11 is a cross-sectional view along the line C-C. As shown in (a) to (e) in Fig. 11, the box-type structure is composed of a combination of five shielding plates 41, 42, 43, 44, and 45. As shown by (f) to (h) in Fig. 11, these shielding plates are abutted and joined to each other via a joining structure formed at the edge portions. One opening is provided at the upper portion of the box-type structure to give a structure in which the joining portions at the edge portions of the shielding plates are arranged to protrude outwards along the periphery of the opening. Accordingly, covering the opening with another shielding plate makes it possible to give a joining structure including end faces contacting each other closely. In addition, it is possible to configure a rectangular parallelepiped box-type structure by combining the box-type structure with another box-type structure through connecting the openings to each other. A shielding plate may be attached to the opening so as to close a part of the opening.

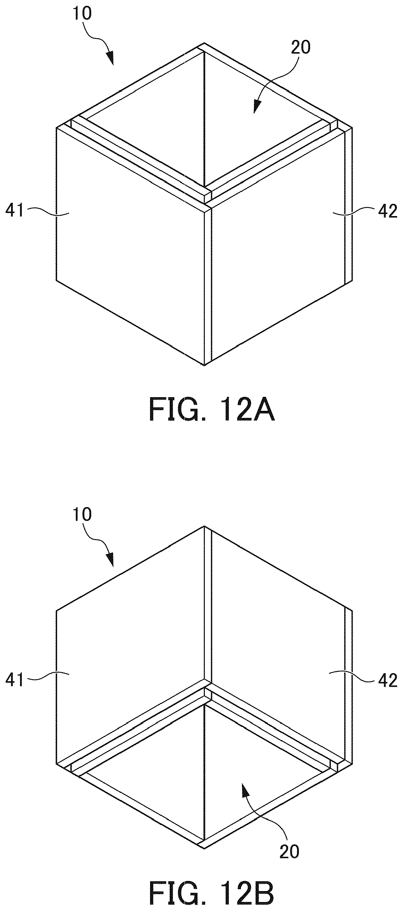

(a) and (b) in Fig. 12 show another embodiment related to the box-type structure to which a shielding adhesive is applied. (a) in Fig. 12 is a perspective view, and (b) in Fig. 12 is a perspective view from another direction. As shown by (a) and (b) in Fig. 12, this is an example including an opening provided at each of the upper side and the lower side of the box-type opening portion. Similar to the box-type structure in Fig. 10, there is provided a structure in which the joining portions at the edge portions of the shielding plates are arranged to protrude outward along the peripheries of the openings. Except for the above, similar to the box-type structure in Fig. 10, the shielding plates 41, 42, 43, and 44 have a structure including the edge portions are abutted and joined via the joining portions.

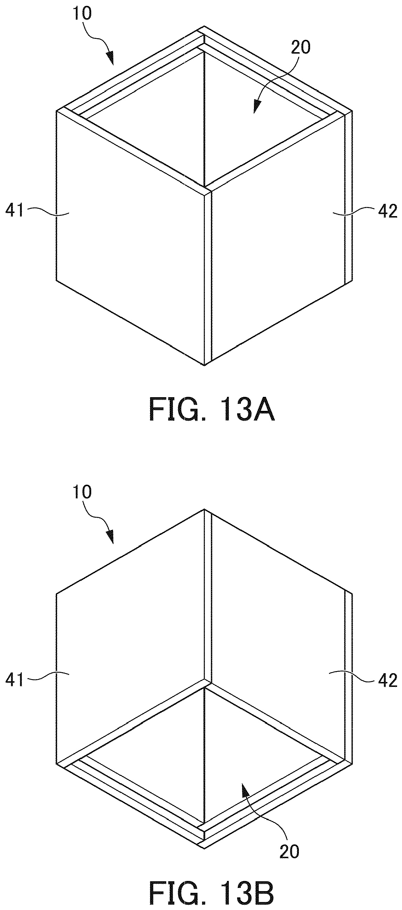

(a) and (b) in Fig. 13 are views illustrating another embodiment related to the box-type structure to which a shielding adhesive is applied. (a) in Fig. 13 is a perspective view, and (b) in Fig. 13 is a perspective view from another direction. As shown by (a) and (b) in Fig. 13, this is an example including an opening provided at each of the upper side and the lower side of the box-type opening portion. Similar to the box-type structure in Fig. 11, there is provided a structure in which the joining portions of the edge portions of the shielding plates are arranged to face inward along the peripheries of the openings. Except for the above, similar to the box-type structure in Fig. 10, the shielding plates 41, 42, 43, and 44 have a structure including the edge portions abutted and joined via the joining portions is provided.

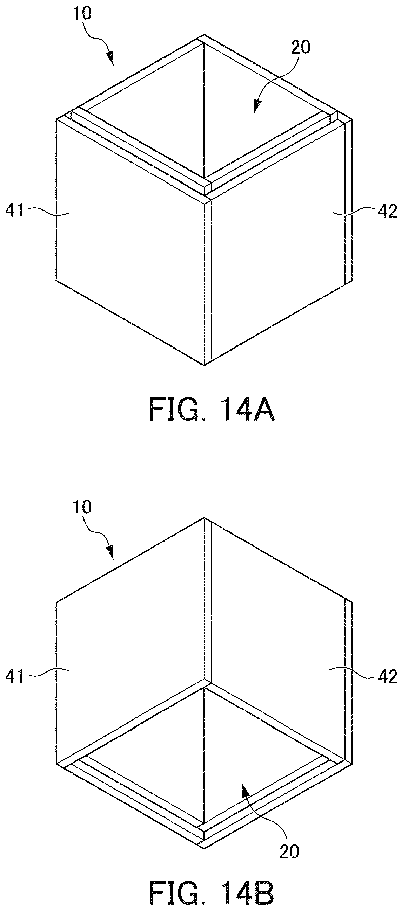

(a) and (b) in Fig. 14 show another embodiment related to the box-type structure to which a shielding adhesive is applied. (a) in Fig. 14 is a perspective view, and (b) in Fig. 14 is a perspective view from another direction. As shown by (a) and (b) in Fig. 14, this is an example including an opening provided at each the upper side and the lower side of the box-type opening portion. Similar to the box-type structure in Fig. 10, there is provided a structure in which the joining portions at the edge portions of the shielding plates are arranged to protrude outward along the periphery of the opening at the upper side. Similar to the box-type structure in Fig. 11, there is provided a structure in which the joining portions at the edge portions of the shielding plates are arranged to face inward along the periphery of the opening at the lower side. Except for the above, similar to the box-type structure in Fig. 10, the shielding plates 41, 42, 43, and 44 have a structure including the edge portions abutted and joined via the joining portions.

The shielding plates shown in Figs. 10 to 14 each have a square plane shape, but the shape is not limited thereto and may be a rectangle as shown in Fig. 15.

### (Lithium fluoride powder material and a lithium fluoride sintered body)

Regarding a material containing lithium fluoride (LiF), Li includes two stable isotopes ⁶Li and ⁷Li. The natural abundance ratios of ⁷Li and ⁶Li are 92.5 atom% and 7.5 atom%, respectively. Since ⁶Li contributes to the shielding of a neutron beam, the neutron beam can be more efficiently shielded by using ⁶LiF containing ⁶Li. The shielding plate made of a lithium fluoride-containing material is preferably a lithium fluoride sintered body prepared by shaping and sintering a lithium fluoride powder to obtain an edge portion structure having a predetermined shape. For the material of the shielding plate, the content of ⁶Li can be adjusted according to the necessary neutron-shielding performance. For example, when high neutron-shielding performance is required, it is possible to select a lithium fluoride powder material having a ⁶Li content of 95 atom% and a LiF purity of 99% or more. Alternatively, it is also possible to use lithium fluoride having an appropriately adjusted content ratio of ⁶Li and ⁷Li.

A lithium fluoride sintered body including ⁶LiF can be obtained without adding a sintering agent or other inorganic compound to form a composite material. Accordingly, the shielding plate made of lithium fluoride can have excellent neutron-shielding performance due to the high purity of the lithium fluoride itself.

### (Lithium fluoride powder material used in shielding adhesive)

The lithium fluoride powder material used in the shielding adhesive can be an equivalent to the lithium fluoride powder used for sintering of the shielding plates. The use of such a lithium fluoride powder allows shielding performance to be obtained with a smaller amount thereof. For example, it is preferable to use a lithium fluoride powder having a ⁶Li content of 95 atom% and a LiF purity of 99% or more.

For the lithium fluoride-containing material according to the invention, the purity of LiF is 99 wt% or more. If the shield material contains a large amount of impurities, the impurities irradiated with a neutron beam might be radiaoactivated to emit gamma rays. LiF itself is not radioactivated even if irradiated with a neutron beam. Accordingly, regarding the lithium fluoride-containing material according to the present embodiment, if the purity of the lithium fluoride itself is low or a sintering agent or other inorganic compound, i.e., a composite material, is mixed, such impurities or mixed components may be radioactivated and emit gamma rays. Thus, the use of lithium fluoride having a high purity is desirable.

Examples of methods for manufacturing a lithium fluoride product include a single crystal growth method, a solidifying method from a melt, and a sintering method. However, the sintering method is preferable because this method makes it possibly to supply a stable-quality product at a low cost.

The single crystal growth method requires high control accuracy during manufacturing, resulting in low quality stability and a significantly high product price. In addition, the resulting single-crystal body has problems such as a high cost for processing into a predetermined shape, cleavability, and the easy occurrence of cracks during processing. The solidifying method from a melt requires strict temperature control during cooling and requires cooling for a long time, resulting in difficulty in obtaining a solid substance having a relatively large size and is homogeneous and defect-free throughout the substance.

The sintered body of lithium fluoride (hereinafter may be also referred to as "LiF sintered body") preferably has a relative density of 86% or more and 92% or less. In the present embodiment, the relative density is a value obtained by dividing the density of a sintered body by the theoretical density of LiF (2.64 g/cm³) and multiplying the result by 100. The lithium fluoride sintered body having a relative density within the above range has the advantage that swelling and the occurrence of voids and cracks during sintering are suppressed to provide excellent machinability. The LiF sintered body is not highly-densified, resulting in the advantage of excellent machinability.

If the relative density is too low, there is a risk that the LiF sintered body does not have sufficient neutron-shielding performance. In addition, if the relative density is too low, there is a concern that the ratio of voids inside the sintered body is high, resulting in an inferior mechanical strength.

In contrast, if the relative density is too high, the LiF sintered body has sufficient neutron-shielding performance. However, there is a concern that the sintered body is highly densified such that the processing of the sintered body may cause cracks or the like due to a released residual stress inside the material.

The thickness of the LiF sintered body is not particularly limited as long as a neutron beam can be suitably blocked. Specifically, the thickness of the LiF sintered body is preferably 2 mm or more and more preferably 3 mm or more from the viewpoint of the mechanical strength of the sintered body and workability during edge portion processing.

The upper limit of the thickness of the LiF sintered body is not particularly limited. From the viewpoint of reducing the size and weight of the shielding plate, a thinner LiF sintered body is preferred within a range capable of suitably shielding a neutron beam. Specifically, the thickness of the LiF sintered body is preferably 8 mm or less and more preferably 5 mm or less.

### (Method for manufacturing LiF sintered body)

The method for manufacturing the LiF sintered body , which can be used with the present invention, includes a pressing step of pressing a LiF composition containing a LiF powder and an organic shaping agent to prepare a pressed body, and a firing step of firing the pressed body at 630°C or more and 830°C or less. Prior to the firing step, for example, a preliminary firing step for degreasing the organic shaping agent may be performed.

### EXAMPLES

The present invention will now be described in more detail using examples. The present invention is not limited to these descriptions.

### (Manufacturing of a box-type structure, which can be used with the present invention)

Base plates of a lithium fluoride (LiF) sintered body having a length of 80 mm, a width of 40 mm, and a thickness of 5 mm were produced. This sintered body had a relative density within a range of 88.9% to 91.3%. A shielding plate 1A and a shielding plate 2A each having an edge portion structure with a predetermined shape were produced by performing edge portion processing for forming a step having a length of about 2.5 mm along the peripheries of the base plates. The shielding plates 1A and 2A were each provided with a step at each of the three peripheral edges. In addition, base plates having a length of 80 mm, a width of 40 mm, and a thickness of 5 mm were produced and subjected to edge portion processing for forming a step having a length of about 2.5 mm at each of the four peripheral edges to produce shielding plates 3A and 3B. Similarly, a base plate having a length of 80 mm, a width of 45 mm, and a thickness of 5 mm was produced and subjected to edge portion processing to form a step having a length of about 2.5 mm at each of the four edges to produce a shielding plate 4A. Furthermore, a base plate having a length of 80 mm, a width of 25 mm, and a thickness of 5 mm was similarly produced and subjected to edge portion processing to form a step having a length of about 2.5 mm at each of the three sides to produce a shielding plate 4B.

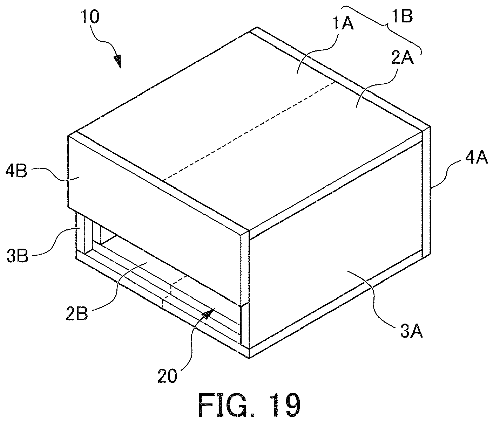

Subsequently, the shielding plate 1A and the shielding plate 2A were abutted against each other such that two were joined to each other at the edge portions of the side faces where no steps were provided in the longitudinal direction, to produce a shielding plate 1B and a shielding plate 2B each having a length of 80 mm and a width of 80 mm. In the abutting and joining, a shielding adhesive was used for fixation. These shielding plates 1B, 2B, 3A, 3B, 4A, and 4B were combined to assemble a box-type structure as shown in Fig. 19.

### (Adhesive containing lithium fluoride according to the present invention)

A lithium fluoride powder (LiF powder) having a neutron-shielding function with a ⁶Li content of 95 atom% was mixed with commercially available adhesives to prepare shielding adhesive samples, and performance of the shielding adhesives was verified. The types of the adhesives used (S1 to S5) and the mixing ratios (mass ratios) of the LiF powder and each adhesive were as shown in Tables 1 to 3. Table 1 shows data for the case where a LiF powder having an average particle size of 3.8 µm was used. Table 2 shows data for the case where a LiF powder having an average particle size of 2.4 µm was used. Table 3 shows data for the case where a LiF powder having an average particle size of 5.2 µm was used.

Two test plates each having a length of 40 mm, a width of 20 mm, and a thickness of 5 mm and made of a LiF sintered body were prepared. The edge portions of the test plates were flat and did not have steps. About 30 mg of an adhesive for the test was applied to the 40 mm length end faces of the test plates, and the test plates were abutted and joined to each other. The excess adhesive overflowing from the gap was removed, followed by temporary fixing and being left to cure until it reached the practical strength described in the respective instruction manuals.

The performance of each adhesive was evaluated from the viewpoints of kneadability, workability, physical properties after curing (stiffness and heat resistance), and shielding properties as shown in Tables 1 to 3. Then, the performance was ranked in accordance with each evaluation criteria of good (A), moderate (B), and unsuitable (C) according to the degree thereof. The results are shown in Tables 1 to 3 with the symbols "A", "B", and "C". The symbol "-" in Tables 1 to 3 indicates performances that were not evaluated. Samples that were rated C for kneadability or workability were not evaluated for physical properties after curing.

Kneadability is a performance to evaluate the degree of uniformity in the paste-like mixture prepared by kneading an adhesive and a LiF powder at a predetermined mixing ration. For Kneadability, a lower viscosity of the adhesive or a lower mixing ratio of the LiF powder/adhesive was determined to be better.

### <Method for measuring average particle size>

The particle size distribution of the LiF powder was measured using a Microtrac MT3300 manufactured by Nikkiso Co., Ltd. under the following conditions: particle refractive index: 1.39, solvent (water) refractive index: 1.33, particle permeability: transmission, particle shape: non-spherical, external dispersion: none, and internal dispersion: 0 to 3 minutes. The 50% accumulation diameter µm obtained by measurement was defined as the average particle size.

### <Kneading method>

In the case of a one-liquid curing type adhesive, a predetermined amount (200 mg) of the adhesive was weighed on a resin sheet using a microspatula. A predetermined amount (200 mg) of the lithium fluoride powder separately-weighed using a medicine wrapping paper was transferred to a spot near the dropped adhesive, and they were then kneaded with a small resin spatula until become uniform. The kneaded product was applied to the joining portion of a lithium fluoride sintered body.

In the case of a two-liquid curing type adhesive, a predetermined amount (100 mg) of each of the adhesive main agent and the adhesive curing agent were separately dropped onto a resin sheet at an interval of several cm using a microspatula. A predetermined amount (100 mg) of lithium fluoride powder separately-weighed using medicine wrapping paper was transferred to a spot near the adhesive main agent and a spot near the adhesive curing agent. Subsequently, the mixture of the adhesive main agent and the lithium fluoride powder and the mixture of the adhesive curing agent and the lithium fluoride powder were then separately kneaded using a spatula similar to the above until become uniform. Then, the mixtures were combined and kneaded together using a spatula similar to the above until becomes uniform. The kneaded product was applied to the joining portion of a lithium fluoride sintered body.

Workability was evaluated according to the balance between appropriate working time and curing time When the LiF powder and the adhesive are kneaded, if the curing of the adhesive proceeds too quickly, uniform mixing is difficult. In contrast, if the adhesive has a low viscosity and takes a long time to cure, it is necessary to fix the joining material with a jig or the like for a long time. In addition, filling property and shape retention property were also considered from the view point of the degree how easily the mixture fills the gap at the joining site and how much the mixture flow out to reduce.

The physical properties after curing were evaluated for stiffness and heat resistance. Regarding the stiffness, test pieces having a length of 40 mm, a width of 5 mm, and thickness of 5 mm were each prepared by cutting a joined sintered body in a direction perpendicular to the joining line into a strip having a width of about 5 mm. A force was applied with a hand so as to fold the test piece in order to verify the presence or absence of interfacial peeling or cohesive fractures at the joining portion and the presence or absence of fractures at the sintered body portion. A test piece that was fractured at the sintered body portion was rated 'A'. A test piece that was fractured at the joining portion, although it would not cause a problem in normal use, was rated as 'B'.

Regarding heat resistance, the test pieces were heated at 100°C for 1 hour with an electric dryer. A test piece wherein the joining portion was not deformed by heating and the strength before heating was mostly maintained was rated 'A'.

Regarding shielding properties, since the required shielding properties vary depending on the purpose, the amounts of LiF contained in the shielding adhesives used were listed as relative values in Table 1.

The results of evaluation from the above-described viewpoints are shown in Tables 1 to 3. Table 1 shows the results for the case where a LiF powder having an average particle size of 3.8 µm was used. As shown in Table 1, it was understood that the two-liquid curing type epoxy adhesives used in sample No.s 1 to 3 and 5 to 7 were preferable when comprehensively evaluated in regard to kneadability, workability, and physical properties after curing. It can be said that when the application area to which a shielding adhesive is applied is small, a fast curing type of two-liquid curing type epoxy is preferred, and that when the application number and the application area are large, a two-liquid curing type epoxy is preferred.

In addition, even if the mixing ratio of the LiF powder was 30% or more, satisfactory results were obtained. Samples No.s 9 to 13 are examples wherein a two-liquid type epoxy/modified silicone adhesive was used. The kneadability and the balance between working time and curing time in workability were good. However, under conditions of a low mixing ratio, since the viscosity was low, the filling and shape retention properties were somewhat insufficient, and the stiffness after curing was also somewhat inferior compared to Nos. 1 to 3 and 5 to 7.

The one-liquid type modified silicone used in sample Nos. 15 and 16 was inadequate in its kneadability and workability. Accordingly, the physical properties after joining were not evaluated.

Table 2 shows the results for the case where a LiF powder having an average particle size of 2.4 µm was used. As shown in Table 2, it was understood that the two-liquid curing type epoxy adhesives used in sample No.s 1 to 3 and 5 to 7 were preferable when comprehensively evaluated in regard to kneadability, workability, and physical properties after curing. It can be said that when the application area to which a shielding adhesive is applied is small, a fast curing type of two-liquid curing type epoxy is preferred, and that when the application number and the application area are large, a two-liquid curing type epoxy is preferred.

In addition, even if the mixing ratio of the LiF powder was 30% or more, satisfactory results were obtained. Sample No.s 9 to 13 are examples of using a two-liquid type epoxy/modified silicone adhesive. The kneadability and the balance between working time and curing time in the workability were good. However, under conditions of a low mixing ratio, since the viscosity was low, the filling and shape retention properties were somewhat insufficient, and the stiffness after curing was also somewhat inferior compared to No.s 1 to 3 and 5 to 7.

Since the one-liquid type modified silicone used in sample No.s 15 and 16 was inadequate in its kneadability and workability, the physical properties after joining were not evaluated.

Table 3 shows the results for the case where a LiF powder having an average particle size of 5.2 µm was used. As shown in Table 3, it was understood that the two-liquid curing type epoxy adhesives according to the present invention, and used in sample No.s 1 to 4 and 6 to 9 were preferable when comprehensively evaluated in regard to kneadability, workability, and physical properties after curing. It can be said that when the application area to which a shielding adhesive is applied is small, a fast curing and two-liquid curing type epoxy is preferred, and that when the application number and the application area are large, a two-liquid curing type epoxy is preferred.

In addition, even if the mixing ratio of the LiF powder was 30% or more, satisfactory results were obtained. Sample No.s 11 to 15 are examples wherein a two-liquid type epoxy/modified silicone adhesive was used. The kneadability and the balance between working time and curing time in workability were good. However, under conditions of a low mixing ratio, since the viscosity was low, the filling and shape retention properties were somewhat insufficient, and the stiffness after curing was also somewhat inferior compared to No.s 1 to 3 and 6 to 9.

Since the one-liquid type modified silicone, which is not part of the present invention, used in sample No.s 17 and 18 was inadequate in its kneadability and workability, the physical properties after joining were not evaluated.

**[Table 1]**

| Sample | Adhesive | | LiF/adhesive mixing ratio (mass ratio) | Performance of adhesive | | | | | |
|---|---|---|---|---|---|---|---|---|---|
| | | | | Kneadability | Workability | | Physical properties after curing | | Shielding performance LiF wt% |
| | Product | Type | | | Working time/ curing time | Filling and shape retention properties | Stiffness | Heat resistance | |
| 1 | S1 | Two-liquid curing type epoxy | 20/80 | A | A | B | A | A | 20 |
| 2 | | | 30/70 | A | A | A | A | A | 30 |
| 3 | | | 50/50 | B | A | A | A | A | 50 |
| 4 | | | 60/40 | C | C | - | - | - | - |
| 5 | S2 | Two-liquid curing type epoxy Fast curing type | 20/80 | A | A | B | A | A | 20 |
| 6 | | | 30/70 | A | A | A | A | A | 30 |
| 7 | | | 50/50 | A | B | A | A | A | 50 |
| 8 | | | 60/40 | C | C | - | - | - | - |
| 9 | S3 | Two-liquid curing type epoxy Epoxy resin/ modified silicone | 20/80 | A | A | C | B | A | 20 |
| 10 | | | 30/70 | A | A | B | B | A | 30 |
| 11 | | | 50/50 | A | A | A | A | A | 50 |
| 12 | | | 60/40 | A | A | A | A | A | 60 |
| 13 | | | 67/33 | B | B | A | A | A | 67 |
| 14 | | | 70/30 | C | C | - | - | - | - |
| 15 | S4 | One-liquid type specific modified silicone | 30/70 | C | C | - | - | - | - |
| 16 | S5 | One-liquid type specific modified silicone Fast curing type | 30/70 | C | C | - | - | - | - |

| | | | | | | | | | |
|---|---|---|---|---|---|---|---|---|---|
| S1:Araldite (registered trademark) Standard (manufactured by Huntsman Japan KK, two-liquid curing type epoxy adhesive, epoxy resin/modified polyamine) S2:Araldite (registered trademark) Rapid (manufactured by Huntsman Japan KK, two-liquid curing type epoxy adhesive, fast curing type, epoxy resin/modified polythiol) S3:EP001N (manufactured by Cemedine Co., Ltd., two-liquid curing type epoxy adhesive, epoxy resin/modified silicone) S4:Super X Clear (manufactured by Cemedine Co., Ltd., one-liquid type specific modified silicone polymer) S5:Super X2 Clear (manufactured by Cemedine Co., Ltd., one-liquid type specific modified silicone polymer) | | | | | | | | | |

**[Table 2]**

| Sample | Adhesive | | LiF/adhesive mixing ratio (mass ratio) | Performance of adhesive | | | | |
|---|---|---|---|---|---|---|---|---|
| | | | | Kneadability | Workability | | Physical properties after curing | |
| | Product | Type | | | Working time/ curing time | Filling and shape retention properties | Stiffness | Heat resistance |
| 1 | S1 | Two-liquid curing type epoxy | 20/80 | A | A | B | A | A |
| 2 | | | 30/70 | A | A | A | A | A |
| 3 | | | 50/50 | B | B | A | A | A |
| 4 | | | 60/40 | C | C | - | - | - |
| 5 | S2 | Two-liquid curing type epoxy Fast curing type | 20/80 | A | A | B | A | A |
| 6 | | | 30/70 | A | A | A | A | A |
| 7 | | | 50/50 | A | B | A | A | A |
| 8 | | | 60/40 | C | C | - | - | - |
| 9 | S3 | Two-liquid curing type epoxy Epoxy resin/ modified silicone | 20/80 | A | A | C | B | A |
| 10 | | | 30/70 | A | A | B | B | A |
| 11 | | | 50/50 | A | A | A | A | A |
| 12 | | | 60/40 | A | A | A | A | A |
| 13 | | | 67/33 | B | B | A | A | A |
| 14 | | | 70/30 | C | C | - | - | - |
| 15 | S4 | One-liquid type specific modified silicone | 30/70 | C | C | - | - | - |
| 16 | S5 | One-liquid type specific modified silicone Fast curing type | 30/70 | C | C | - | - | - |

| | | | | | | | | |
|---|---|---|---|---|---|---|---|---|
| S1:Araldite (registered trademark) Standard (manufactured by Huntsman Japan KK, two-liquid curing type epoxy adhesive, epoxy resin/modified polyamine) S2:Araldite (registered trademark) Rapid (manufactured by Huntsman Japan KK, two-liquid curing type epoxy adhesive, fast curing type, epoxy resin/modified polythiol) S3:EP001N (manufactured by Cemedine Co., Ltd., two-liquid curing type epoxy adhesive, epoxy resin/modified silicone) S4:Super X Clear (manufactured by Cemedine Co., Ltd., one-liquid type specific modified silicone polymer) S5:Super X2 Clear (manufactured by Cemedine Co., Ltd., one-liquid type specific modified silicone polymer) | | | | | | | | |

**Table 3**

| Sample | Adhesive | | LiF/adhesive mixing ratio (mass ratio) | Performance of adhesive | | | | |
|---|---|---|---|---|---|---|---|---|
| | | | | Kneadability | Workability | | Physical properties after curing | |
| | Product | Type | | | Working time/ curing time | Filling and shape retention properties | Stiffness | Heat resistance |
| 1 | S1 | Two-liquid curing type epoxy | 20/80 | A | A | B | A | A |
| 2 | | | 30/70 | A | A | A | A | A |
| 3 | | | 50/50 | B | A | A | A | A |
| 4 | | | 60/40 | B | B | A | A | A |
| 5 | | | 67/33 | C | C | - | - | - |
| 6 | S2 | Two-liquid curing type epoxy Fast curing type | 20/80 | A | A | B | A | A |
| 7 | | | 30/70 | A | A | A | A | A |
| 8 | | | 50/50 | A | A | A | A | A |
| 9 | | | 60/40 | A | B | A | A | A |
| 10 | | | 67/33 | C | C | - | - | - |
| 11 | S3 | Two-liquid curing type epoxy Epoxy resin/ modified silicone | 20/80 | A | A | C | B | A |
| 12 | | | 30/70 | A | A | B | B | A |
| 13 | | | 50/50 | A | A | A | A | A |
| 14 | | | 60/40 | A | A | A | A | A |
| 15 | | | 67/33 | B | B | A | A | A |
| 16 | | | 70/30 | C | C | - | - | - |
| 17 | S4 | One-liquid type specific modified silicone | 30/70 | C | C | - | - | - |
| 18 | S5 | One-liquid type specific modified silicone Fast curing type | 30/70 | C | C | - | - | - |

| | | | | | | | | |
|---|---|---|---|---|---|---|---|---|
| S1:Araldite (registered trademark) Standard (manufactured by Huntsman Japan KK, two-liquid curing type epoxy adhesive, epoxy resin/modified polyamine) S2:Araldite (registered trademark) Rapid (manufactured by Huntsman Japan KK, two-liquid curing type epoxy adhesive, fast curing type, epoxy resin/modified polythiol) S3:EP001N (manufactured by Cemedine Co., Ltd., two-liquid curing type epoxy adhesive, epoxy resin/modified silicone) S4:Super X Clear (manufactured by Cemedine Co., Ltd., one-liquid type specific modified silicone polymer) S5:Super X2 Clear (manufactured by Cemedine Co., Ltd., one-liquid type specific modified silicone polymer) | | | | | | | | |

- 20: opening
- 21: joining structure
- 22, 23, 24, 25, 26, 27, 28: shielding plate
- 29: chipped off portion
- 31: upper side of shielding plate
- 32: lower side of shielding plate
- 33: step
- 41, 42, 43, 44, 45, 46: shielding plate
- 51, 52, 53, 54, 55, 56: shielding plate

## Claims

1. A shielding adhesive having neutron-shielding performance, wherein the shielding adhesive contains a lithium fluoride powder having a lithium fluoride purity of 99 wt% or more, **characterized in that** the shielding adhesive is a two-liquid curing type adhesive the main component of which is an epoxy resin.

2. The shielding adhesive according to claim 1, wherein the lithium fluoride powder has an average particle size of 2.4 µm or more and 5.2 µm or less.

3. The shielding adhesive according to claim 2, wherein the shielding adhesive contains the lithium fluoride powder in an amount of 30 wt% or more and less than 60 wt%.

4. The shielding adhesive according to claims 1 or 2, wherein the shielding adhesive further contains a modified silicone resin as a curing agent.

5. The shielding adhesive according to claim 4, wherein the shielding adhesive contains the lithium fluoride powder in an amount of 30 wt% or more and 67 wt% or less.

6. Use of a shielding adhesive according to any one of claims 1 to 5 for repairing a defective part of shielding plates (1, 2, 3, 4) made of a lithium fluoride sintered body or for filling a gap at a joining portion of the shielding plates (1, 2, 3, 4).

## Patentansprüche

1. Abschirmender Klebstoff mit Neutronen-abschirmender Eigenschaft, wobei der abschirmende Klebstoff ein Lithiumfluorid-Pulver mit einer Reinheit an Lithiumfluorid von 99 Gew.-% oder mehr enthält, **dadurch gekennzeichnet, dass** der abschirmende Klebstoff vom Klebstofftyp der Zweiflüssigkeitshärtung ist, wobei dessen Hauptkomponente ein Epoxidharz ist.

2. Abschirmender Klebstoff nach Anspruch 1, wobei das Lithiumfluorid-Pulver eine durchschnittliche Partikelgröße von 2,4 µm oder mehr und 5,2 µm oder weniger aufweist.

3. Abschirmender Klebstoff nach Anspruch 2, wobei der abschirmende Klebstoff das Lithiumfluorid-Pulver in einer Menge von 30 Gew.-% oder mehr und weniger als 60 Gew.-% enthält.

4. Abschirmender Klebstoff nach den Ansprüchen 1 oder 2, wobei der abschirmende Klebstoff ferner ein modifiziertes Silikon-Harz als Härtemittel enthält.

5. Abschirmender Klebstoff nach Anspruch 4, wobei der abschirmende Klebstoff das Lithiumfluoridpulver in einer Menge von 30 Gew.-% oder mehr und 67 Gew.-% oder weniger enthält.

6. Verwendung eines abschirmenden Klebstoffs nach einem der Ansprüche 1 bis 5 zur Reparatur eines beschädigten Teils von abschirmenden Platten (1, 2, 3, 4), hergestellt aus einem gesinterten Körper aus Lithiumfluorid, oder zum Füllen einer Lücke in einem Verbindungsabschnitt der abschirmenden Platten (1, 2, 3, 4).

## Revendications

1. Adhésif de blindage ayant une performance de blindage neutronique, dans lequel l'adhésif de blindage contient une poudre de fluorure de lithium ayant une pureté de fluorure de lithium de 99 % en poids ou plus, **caractérisé en ce que** l'adhésif de blindage est un adhésif de type durcissement à deux liquides dont le composant principal est une résine époxy.

2. Adhésif de blindage selon la revendication 1, dans lequel la poudre de fluorure de lithium a une taille de particules moyenne de 2,4 µm ou plus et de 5,2 µm ou moins.

3. Adhésif de blindage selon la revendication 2, dans lequel l'adhésif de blindage contient la poudre de fluorure de lithium en une quantité de 30 % en poids ou plus et de moins de 60 % en poids.

4. Adhésif de blindage selon les revendications 1 ou 2, dans lequel l'adhésif de blindage contient en outre une résine de silicone modifiée en tant qu'agent de durcissement.

5. Adhésif de blindage selon la revendication 4, dans lequel l'adhésif de blindage contient la poudre de fluorure de lithium en une quantité de 30 % en poids ou plus et de 67 % en poids ou moins.

6. Utilisation d'un adhésif de blindage selon l'une quelconque des revendications 1 à 5 pour la réparation d'une partie défectueuse de plaques de blindage (1, 2, 3, 4) formées d'un corps fritté de fluorure de lithium ou pour remplir un espace au niveau d'une partie de jonction des plaques blindage (1, 2, 3, 4).
